# EUROPEAN PATENT SPECIFICATION

(11) **EP 2 023 988 B1**
(45) Date of publication and mention of the grant of the patent: **21.11.2018**
(21) Application number: 07725330.0
(22) Date of filing: 16.05.2007
(51) Int. Cl.: A61M 15/00

(54) **INHALER**
INHALATOR
INHALATEUR

(30) Priority: 18.05.2006 DE 102006023662
(43) Date of publication of application: 18.02.2009
(73) Proprietor: Boehringer Ingelheim International GmbH, 55216 Ingelheim am Rhein (DE); Vectura Delivery Devices Limited, Chippenham Wiltshire SN14 6FH (GB)
(72) Inventor: THOEMMES, Ralf, 47877 Willich (DE); BESSELER, Jens, 44319 Dortmund (DE); DONKER, Birgit, 44357 Dortmund (DE); FRENTZEL-BEYME, Jessica, 44803 Bochum (DE); Eason, Stephen William, Diss Norfolk IP22 1RX (GB); Clarke, Roger William, Cambridge CB4 9EZ (GB)
(74) Representative: Simon, Elke Anna Maria
(86) International application number: PCT/EP2007/004416
(87) International publication number: WO 2007/134792

(56) References cited:
- WO-A-01/26720
- WO-A-03/035508
- WO-A-03/061743
- WO-A-2005/014089
- WO-A-2005/049121
- WO-A-2006/079746
- DE-A1- 4 106 379

## Description

The present invention relates to an inhaler.

The present invention relates to the delivery and atomisation of a formulation for inhalation or for other medical or therapeutic purposes. Preferably the present invention relates to the delivery of medical, pharmaceutical and/or therapeutic formulations which in particular contain or consist of at least one active substance. During atomisation an aerosol or a spray cloud is produced having, particularly for inhalation, very fine, solid and/or liquid particles, preferably in the range from 1 to 10 µm.

The formulation is a powder in this case. Particularly preferably, the invention therefore relates to a powder inhaler. Otherwise, the term "formulation" could also include liquids, while the term "liquid" is to be understood in the broad sense as including *inter alia* solutions, suspension, suslutions (mixture of solution and suspension), dispersions, mixtures thereof or the like.

The present invention relates to an inhaler for delivering a powdered formulation from a reservoir such as a blister strip or other band-shaped carrier, having a plurality of receptacles or blister pouches, each of which contains one dose of the formulation. The term "inhaler" is therefore preferably to be understood generally as including other atomisers or dispensers for delivering a pre-metered formulation.

DE 41 06 379 A1 discloses an inhaler having a coiled blister strip. Blister pouches of the blister strip are each filled with a dose of a powdered medicament and are opened one after another for inhalation by peeling or pulling off a cover.

WO 03/061743 A1 discloses a medical dispenser for use with (peelable) blister strips, each having multiple distinct medicament dose portions carried thereby. The mechanism for dispensing the dose portions comprises a receiving station for receiving the two carriers, a release (comprising means for peeling apart the blister strip) for releasing the distinct dose portion from each of the blister strips on receipt thereof by the receiving station, an outlet positioned to be in communication with distinct dose portions releasable by said release and an indexer for individually indexing the distinct dose portions of each of the two blister strips. Disclosed are devices in which the two blister strips and their respective indexers are arranged mirror-image like with the respective blister strips being guided along the respective insides (left and right) of the housing of the dispenser.

WO 2005/049121 A1 discloses a device with an endless chain of powder filled capsules which are conveyed along a track which is mostly arranged along the inner wall of the device. Embodiments are disclosed wherein the capsules are either unlinked or chain-linked to each other. The chain-linking of the capsules comes with a simplified conveying mechanism in which the capsule chain can be advanced by a single sprocket, a spindle of which is rotatably connected to a knob which is manually rotatable.

Object of the present invention is to provide an improved inhaler which while being simple in construction accommodates a preferably endless band-shaped carrier or blister strip which is optimum particularly in terms of its compactness and/or ease of operation, and methods of delivering a powdered formulation, in particular permitting simple and/or reliable operation and/or delivery while using a simple, inexpensive construction.

The above object is achieved by means of an inhaler according to claim 1. Advantageous further features are recited in the subsidiary claims.

According to a first aspect of the present invention it is envisaged that the carrier extends over a circumferential angle of the inhaler of less than 360°, is guided between two deflectors each of substantially constant curvature, extends solely in an annular segment of the inhaler and/or extends with one of two portions connecting the deflectors exclusively along a circumferential or outer wall of the inhaler. These measures enable the carrier to extend as a simple loop which has the minimum possible curvature and is accordingly relatively easy to move or convey. This results in a simple but nevertheless compact construction.

The carrier is constructed as a band and/or blister strip. The receptacles are preferably formed by blister pouches. This allows simple and inexpensive manufacture.

According to a second aspect of the present invention which can also be implemented independently, the inhaler has a planet gear and/or a conveyor device having a plurality of wheels, particularly planet wheels, for stepwise advancing and/or deflecting of the carrier. The wheels are of the same diameter, are arranged on a common radius, can be driven directly or indirectly by common drive means, particularly a sun wheel or the like, and/or have the same direction of rotation. This contributes to the desired easy action of the carrier while retaining a simple and compact construction for the inhaler.

According to another aspect of the present invention which can also be implemented independently, the inhaler 1 has a conveyor device with a gear, wherein free mobility is provided in one direction of rotation. Particularly preferably, the free rotation is integrated in the sun wheel of a planet gear. This allows a simple compact construction.

According to another aspect of the present invention which can also be implemented independently, the carrier or blister strip is movable transversely of the direction of advance in order to be able to bring the receptacles containing a dose of the formulation individually up to a preferably fixed removal device and/or open said receptacles. This allows a compact simple construction and/or optimum arrangement of the removal device in the region of a mouthpiece preferably provided in the inhaler.

In another aspect of the present invention, the preferably substantially round housing of the inhaler is flattened or indented on one side, the side being formed in particular by a housing section and a section of an openable mouthpiece cover. This allows simple, intuitive operation.

In another aspect of the present invention, the carrier is moved counter to the direction of conveying, towards a travel limiter formed in particular by a non-return device, for the purpose of accurately positioning its receptacles or blister pouches. Thus very precise positioning can be achieved in a very simple manner. In particular, the travel limiter is formed by a non-return device of a gear that moves the carrier along. However, the non-return device may theoretically also act directly on the carrier or blister strip.

According to another aspect of the present invention, during opening of a cover of a mouthpiece of the inhaler, only the carrier or the respective receptacle for dispensing the next dose is opened and the carrier is only moved on when the cover is closed. In this way malfunctions particularly when only partly opening the cover are at least substantially ruled out in a simple manner.

Further aspects, features, properties and advantages of the present invention will become apparent from the claims and following description of preferred embodiments with reference to the drawings. In the drawings:
- Fig. 1: is a schematic view of an inhaler according to a first embodiment in the open state;
- Fig. 2: is a perspective view of a guide portion of the inhaler;
- Fig. 3: is a section through a part of the inhaler in the region of a mouth-piece;
- Fig. 4: is an external view of the inhaler; and
- Fig. 5: is a schematic view of an inhaler according to a second embodiment in the open position and in schematic section.

In the Figures, the same reference numerals have been used for identical or similar parts and components; in particular, similar or corresponding advantages and/or properties are obtained even if the associated description is not repeated.

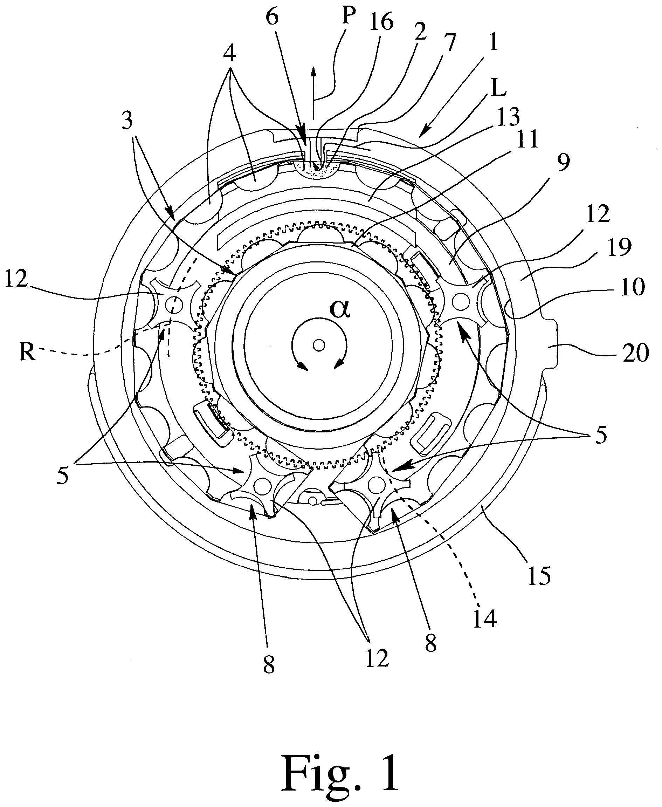

Fig. 1 shows in a highly schematic representation a proposed inhaler 1 according to a first embodiment, in a cut-away or open state without a lid or cover.

The inhaler 1 serves to deliver a powdered formulation 2 in the sense described hereinbefore from a band-shaped carrier, particularly a blister strip 3, having a plurality of receptacles, especially blister pouches 4, each of which directly contains a dose of the, in particular, loose formulation 2. The carrier forms a closed loop, i.e. it is of circular or endless construction. For inhalation and particularly during inhalation, preferably one dose of the formulation 2 is taken from a receptacle or blister pouch 4.

The inhaler 1 has a conveyor device 5 for advancing or conveying the carrier stepwise.

The inhaler 1 also has a removing device 6 for individually opening the receptacles and/or removing the doses of formulation 2. In particular the removal device 6 is constructed so that the receptacles can be opened individually and successively from the outside and/or by breathing in while inhaling an air current L of ambient air can be sucked in, in order to deliver the respective dose with the ambient air through an associated mouthpiece 7 of the inhaler 1, as indicated by the arrow P in Fig. 1.

The mouthpiece 7 of the inhaler 1 is preferably of rigid construction and/or is formed on or formed by a housing of the inhaler 1.

Instead of the mouthpiece 7 the inhaler 1 may also have another endpiece, for example for nasal or other routes of administration for delivering the formulation 2. In particular, the inhaler 1 may also be used as a nebuliser for other purposes, e.g. for the eyes. The term "inhaler" should therefore preferably be understood in a correspondingly general sense.

The conveying device 5 preferably has two deflectors 8 for the carrier. In particular, the carrier extends only between the deflectors 8, particularly preferably in such a manner that the maximum possible length of carrier can be accommodated in the inhaler 1 whilst subjecting the carrier to the least possible and/or most uniform possible curvature, and/or so as to achieve easy action with a simple and compact construction of the inhaler 1. These individual aspects are discussed in more detail hereinafter.

The carrier is preferably guided exclusively in an annular segment 9 of the inhaler 1, as shown in Fig. 1. In particular, the inhaler 1 or the annular segment 9 forms a channel for the carrier between an in particular peripheral outer wall 10 and an intermediate or inner wall 11 of the inhaler 1.

The outer wall 10 extends in particular substantially along a circle in the particularly preferred, substantially disc-shaped construction of the inhaler 1. The inner wall 11 is preferably in the form of an outer surface of a cylinder. However, other shapes are also possible.

The deflectors 8 are preferably arranged relatively close together. The carrier preferably extends away from the deflectors 8 in opposite directions.

The carrier extends over a circumferential angle α of the inhaler 1 of at least 270° and/or less than 360°. Thus a spiral or helical configuration and hence a difficult action of the carrier can be avoided.

The carrier is guided between the two deflectors 8 with an at least substantially constant curvature. This contributes in particular to the desired easy action for moving or conveying the carrier.

Portions of the carrier extend between the two deflectors 8. One of these portions - in the embodiment shown, the radially outer portion - preferably extends exclusively along the outer wall 10. This makes optimum use of the space available while in particular achieving an at least substantially constant and/or minimal curvature of the carrier along this portion.

The deflectors 8 deflect the carrier through at least 160°, particularly at least substantially 180°. This contributes to the desired compact structure of the inhaler 1.

The portions of the carrier between the two deflectors 8 are preferably guided at an at least substantially constant spacing from one another. In particular, the radial width of the annual segment 9 or the radial spacing of the outer wall 10 from the inner wall 11 over the preferably arcuate extent of the carrier or the circumferential angle α is constant.

Preferably, the carrier runs exclusively between the two deflectors 8. In particular, the portions between the two deflectors 8 are guided at least substantially along an outer and inner arc, particularly an arc of a circle, as can be seen from Fig. 1.

In particular, the carrier 1 is guided on the inside by means of the preferably circular or cylindrical inner wall 11. On the outside or along the outer wall 10, the carrier is preferably guided by means of the deflectors 8, wheels 12 and/or a guide element 13.

The wheels 12 are preferably all of uniform construction but may if necessary also be of different construction.

At least one wheel 12 is preferably constructed so that the carrier can be conveyed or moved along by interlocking engagement, so that the carrier can be advanced or conveyed onwards step by step to the next receptacle by corresponding stepwise rotation of the wheel 12. This enables the formulation 2 to be removed from the individual receptacles one after another and individually taken for delivery through the removal device 6. Preferably all the wheels 12 are constructed for movement or conveying of the carrier by interlocking engagement.

Particularly preferably, the wheels 12 are constructed accordingly as drive wheels or gears or pinions. Thus, in particular, the receptacles can engage in corresponding recesses in the wheels 12. Alternatively or additionally, the wheels 12 may also engage with teeth, projections or the like into corresponding recesses, openings, interstices or the like in the carrier in order to allow the desired conveying of the carrier by interlocking engagement or in defined manner.

Alternatively or additionally, the wheels 12 or at least some of the wheels 12 may also be formed as rollers or the like and/or may not enter into interlocking engagement with the carrier. Rather, the carrier may if necessary be guided only by frictional engagement or possibly by slipping or sliding. The wheels 12 are then constructed in particular as loose, non-driven rollers or the like.

The deflectors 8 or wheels 12 may theoretically also be formed by sliding elements or other guide means. However, it is particularly preferred that the deflectors 8 should be formed by wheels 12.

In the embodiment shown, two wheels 12 form the deflectors 8. In addition, at least two other wheels 12 are preferably provided for guiding the carrier, particularly the outer portion of the carrier, on or along the outer wall 10. However, these additional wheels 12 may also be omitted entirely. In this case, in particular, corresponding guides, channels or wall portions are provided for adequately guiding the portions of the carrier between the deflectors 8.

The two deflectors 8 are preferably arranged on the side of the inhaler 1 opposite the removal device 6 or mouthpiece 7. Particularly preferably, the carrier is arranged symmetrically with respect to the removal device 6 and/or mouthpiece 7. Alternatively or additionally, this is preferably also true of the arrangement of the wheels 12.

A particular advantage of the proposed guiding of the carrier in the inhaler 1 is that the carrier is only relatively highly curved in the region of the deflectors 8 and, in particular, is only curved in the same direction. In particular, there is no sharp curve in the opposite direction. This contributes to the desired easy action during the conveying or movement of the carrier.

The wheels 12 preferably have the same diameter and in particular are identical in construction. This makes manufacture simple and inexpensive. Particularly preferably, the diameter of the wheels 12 substantially corresponds to the spacing of the portions of the carrier.

The wheels 12 are preferably arranged on a common radius R. In particular, this permits simple driving. Preferably, in fact, the wheels 12 can be driven by common drive means, especially a sun wheel 14, which is only partly shown in Fig. 1. In this case a kind of planet gear is formed, in particular, with the wheels 12. The wheels 12 preferably have teeth in the manner of a gear, to allow a defined movement or conveying of the carrier. Alternatively, the wheels 12 may also be driveable, for example, by means of an encircling belt or the like as a common drive means.

Particularly preferably, the wheels 12 rotate in the same direction in order to move or convey the carrier.

Thus, during the actuation of the conveying device 5 the carrier continues to be advanced or conveyed onwards in a rotary manner, and indeed is advanced stepwise to the next receptacle for the next delivery or inhalation.

The conveying device 5 preferably forms the only drive for moving the carrier.

The mouthpiece 7 or other endpiece preferably has an associated cover 15 which is movable, in particular pivotable, in order to open and/or close the mouthpiece 7. The conveying device 5 and/or removal device 6 is preferably actuated by the opening and/or closing movement of the cover 15. This provides a very simple and, in particular, intuitive method of actuating and using the inhaler 1.

The removal device 6 preferably has a removal element 16 which is constructed in particular as a piercing element, is of fixed design and/or attached to the mouthpiece 7. The removal element 16 is movable relative to the respective receptacle or to the carrier in order to open the respective receptacle, in particular to pierce it, and thereby establish a fluidic connection with the receptacle in question.

Preferably the relative movement is carried out by the carrier being moved by the guide element 13 or in some other way, in particular pushed transversely with respect to its direction of movement or conveying. For this purpose the guide element 13 is correspondingly guided to be movable, particularly slideable, most preferably in the manner of a sled. This direction of movement or sliding is in particular in the radial direction, in the embodiment shown, or in or counter to the direction of delivery or the arrow P.

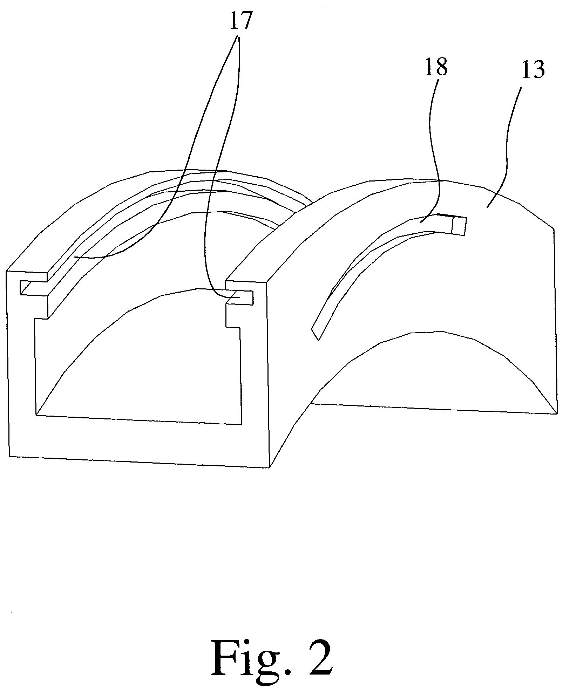

Fig. 2 shows in perspective view a preferred construction of the guide element 13. The guide element 13 guides the carrier preferably by interlocking engagement, e.g. in opposing longitudinal grooves 17 open towards one another. However, other constructional solutions are also possible.

Moreover, Fig. 2 shows a preferably exterior sliding surface, control groove or control curve 18 on the guide element 13. The cover 15 may engage directly or indirectly in this control groove or may engage on this control curve 18, so that the guide element 13 and hence the carrier are moved or displaced in the desired manner relative to the removal element 16 for selectively opening or making contact with the respective receptacle. In particular, a sliding or forcible guide is formed for moving the guide element 13 or the carrier in defined manner as desired. However, other geared solutions or actuations are also possible.

For use, the cover 15 of the inhaler 1 is opened, for example by pivoting or rotating about a central axis of the inhaler 1. This exposes the mouthpiece 7. Before this, at the same time or subsequently, the conveying device 5 is actuated or the carrier is advanced to the next receptacle and/or the next receptacle is opened. This is achieved in particular by means of a suitable geared coupling of the cover 15 with the conveying device 5, particularly preferably with the sun wheel 14.

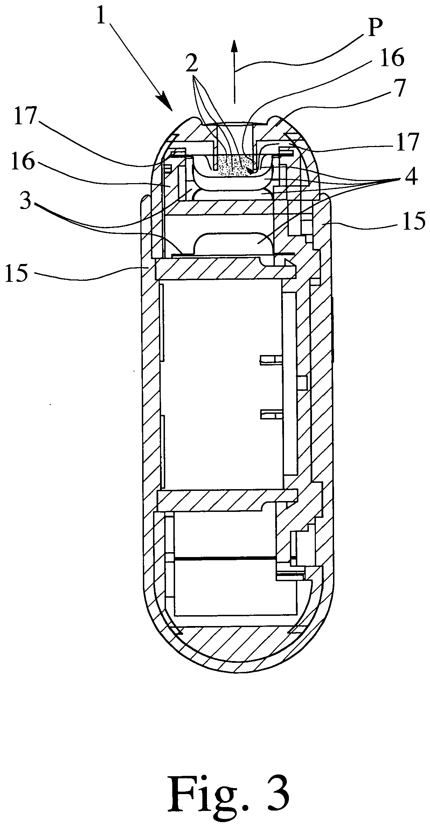

After the carrier has been advanced in the desired manner, and preferably as the opening or pivoting movement of the cover 15 continues, the receptacle intended for the next inhalation, and in particular already positioned relative to or underneath the removal element 16, is opened. To open it, the guide element 13 or the carrier is moved relative to the removal element 16 such that the latter opens the receptacle, particularly engages in a foil cover or covering of the blister pouch 3 and cuts it open, pierces it or tears it open. This state is illustrated in Fig. 1 and in Fig. 3 in a cut away schematic section through part of the mouthpiece 7. In this states the cover 15 is fully open and the inhaler 1 is ready for delivery or inhalation.

According to a particularly preferred alternative feature the carrier is not conveyed further during the opening of the cover 15 but is opened only in the course of the opening movement (in particular, only at the end of the opening movement). In particular, the respective receptacle of the carrier is pierced by the removal element 16. Particularly preferably the opening is carried out by moving the carrier or the corresponding receptacle up to the preferably stationary removal element 16. In this way the opening movement is at an end and the cover 15 in particular is fully open. Now the delivery or inhalation of the dose contained in the open receptacle can take place. Once the delivery or inhalation of the dose has ended the cover 15 is closed again. During the closing movement, first of all in a first phase the removal element 16 and the carrier are moved away from one another, and in particular the carrier is moved away from the removal element 16 again, so that the carrier can be moved onwards. Then in a second phase of the closing movement of the cover 15 the carrier is moved on, so that the next receptacle is already in position or at least substantially correctly positioned. This procedure has the advantage that partial opening of the inhaler 1 or cover 15 starting from the closed position does not lead to any further movement of the carrier.

In the alternative feature described above a freewheeling clutch 22 or a non-return device 28 in particular ensures, as explained hereinafter with reference to a second embodiment, that the carrier does not move back during the opening of the cover 15 or is only moved back slightly for accurate positioning.

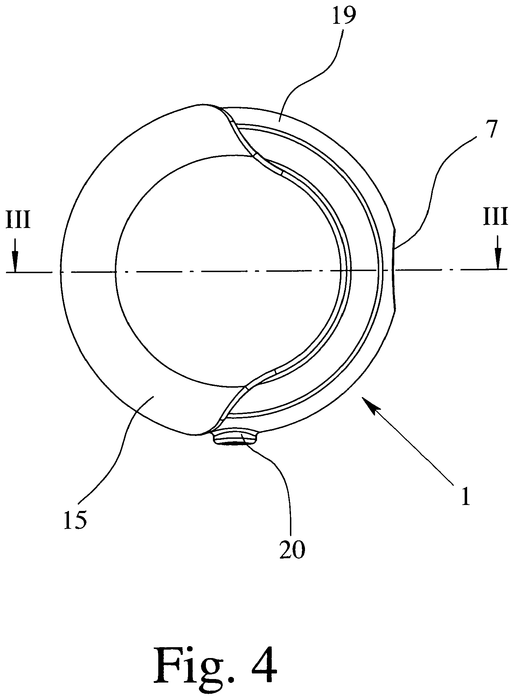

Fig. 4 shows the inhaler 1 in exterior view with the cover 15 open. The cover 15 surrounds the preferably disk-shaped housing 19 of the inhaler 1, in particular in the shape of a sector on both sides and along a circumferential angle or circumferential portion of for example about 120 to 180°. Preferably at least one stop, in this case a radial stop 20, is provided for limiting the pivoting movement, particular both the opening movement and the closing movement.

When a user or patient (not shown) breathes or sucks in air through the mouthpiece 10, the air current L of ambient air is sucked in and conducted through the opened or pierced receptacle in such a way that the formulation 2 contained in the receptacle is delivered by the air current L through the removal element 16 and the adjoining mouthpiece 7 in nebulised form, i.e. as an aerosol cloud or spray missed.

After the receptacle has been emptied and the formulation 2 delivered, the inhaler 1 or the cover 15 can be closed again. To do this, the cover 15 is preferably rotated or pivoted counter to the direction of opening. A corresponding freedom of movement or locking mechanism ensures that the carrier or blister strip 3 is not moved or conveyed in the opposite direction in undesirable manner.

According to the alternative embodiment already described, in addition to or in particular as an alternative to the opening movement, the closing movement may also be used to actuate the conveying device 5 or to advance or further convey the carrier on to the next receptacle.

The inhaler 1 is preferably portable in construction. Particularly preferably, it operates purely mechanically.

The inhaler 1 preferably comprises a counter (not shown) for counting and particularly displaying the receptacles which have already been used or those which have not yet been used.

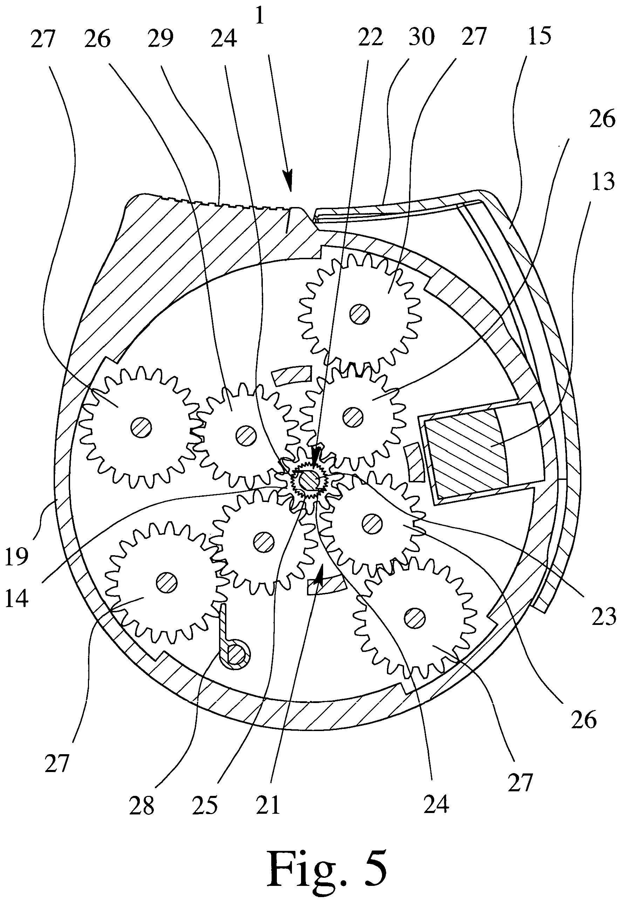

A second embodiment of the proposed inhaler 1 will now be described in more detail with reference to Figure 5. Only major differences from the first embodiment will be described here, in particular, which means that the other remarks and explanations still apply accordingly or in supplementary manner.

In the second embodiment or in the section according to Figure 5, a gear is shown, particularly a gearwheel or planetary gear 21, of the inhaler 1 or of the conveying device 5 for advancing or further conveying the carrier. However, theoretically, any other suitable gear may be used, although the planet gear 21 is particularly preferred.

Preferably, the inhaler 1 or the conveying device 5 is free running 22 in one direction of driving or rotation of the drive train or gear.

Particularly preferably, the free running 22 is associated with a drive wheel or gearwheel, particularly the sun wheel 14, or is arranged or integrated therein. In the embodiment shown, the free running 22 comprises a drive element 23 with at least one preferably tangentially and/or radially projecting finger 24 (most preferably two fingers 24). The drive element 23 engages in one direction of rotation (the anticlockwise direction in the embodiment shown) in interlocking engagement by means of the finger 24 or fingers 24 on the associated drive or gearwheel or sun wheel 14 in order to drive the latter in this direction. In the other direction or rotation the fingers 24 can glide or slip through. In particular, inner teeth 25 are formed on the associated drive or gearwheel or sun wheel 14 on which the fingers 24 can engage. However, other constructional solutions are also possible.

The sun wheel 14 preferably drives associated planet wheels 27 of the planet gear 21 via intermediate gears 26. The intermediate gears 26 are preferably uniformly distributed about the sun wheel 14 in order to achieve a particularly compact structure. Alternatively, the sun wheel 14 may also drive the planet wheels 27 directly.

The planet wheels 27 are preferably arranged coaxially with the wheels 12 for guiding and/or conveying the carrier. Particularly preferably, the planet wheels 27 are mounted about wheels 12, in pairs on a common axle and/or are of integral construction. For example, the planet wheels 27 may be formed on the wheels 12 or vice versa.

The inhaler 1 or the conveying device 5 preferably has a non-return mechanism 28 to safely prevent undesirable reversing of the carrier and/or to form a defined end stop to the movement of the carrier. The non-return mechanism 28 preferably works on the conveying device 5 or its gear, i.e. in the embodiment shown on a planet wheel 27. For example, the non-return mechanism 28 may be formed by an, in particular, elastically biased locking latch or the like which prevents the unwanted backwards rotation and reversing. Alternatively or additionally, the non-return mechanism 28 may also act on the carrier itself.

In the embodiment shown the drive element 23 is preferably coupled in a driving relationship with the cover 15, so that the drive element 23 is rotated at the same time during opening and closing. In one direction of rotation the free wheel clutch 22 locks in place and rotates the sun wheel 14 in order to drive the planet wheels 27 and according convey the carrier onwards by means of the wheels 12. In the other direction of rotation the free wheel clutch 22 comes into effect or rotates or slips through, at least under the effect of the non-return mechanism 28, so that the carrier is no longer moved backwards or at most is moved as far as the stop or the barrier of the non-return mechanism 28. This reversing as far as a defined stop by means of the non-return mechanism 28 can be achieved by corresponding frictional or forceful engagement of the free wheel clutch 22 in the reverse direction and/or it can be used for highly accurate positioning of the carrier.

Preferably, the inhaler 1 is constructed such that the carrier is initially always advanced somewhat too far as it moves on to the next receptacle. Before the receptacle is opened, the carrier is then moved back to the travel limiter or until the non-return mechanism 28 comes into effect. Thus, the receptacle which is to be opened is initially positioned very precisely in the removal position in which opening takes place, particularly by moving it up to the removal element 16.

Particularly preferably, the reversing of the carrier or of the receptacle in the carrier which is to be opened, up to a defined end stop (formed by the non-return mechanism 28), is used in the alternative embodiment already described above (advancing of the carrier only when the cover 15 is shut) so that during the initial opening of the inhaler 1 or of the cover 15 the carrier is moved back to the stop and the receptacle which is to be opened is accurately positioned relative to the removal element 16. The actual opening of the receptacle then takes place in the course of the further opening movement of the cover 15, as already explained.

Generally speaking it should be pointed out that the free wheel clutch 22 may also be produced in some other way. The non-return mechanism 28 can also be formed in some other way. In particular the non-return mechanism 28 may if necessary also act directly on the carrier 2, optionally even directly on the receptacles or blister pouches 4.

It should be noted that the opened covered 15 in turn preferably exposes the mouthpiece 7, although this is not shown in Figure 5.

Figure 5 shows the preferred sled-like guiding of the guide element 13, preferably by means of the housing of the inhaler 1, in purely schematic form.

The inhaler 1 or its housing 19 has in particular a substantially round shape and/or a flattened or indented (concave) side. In the embodiment by way of example, this flattened or indented side is particularly preferably formed by a portion 29 of the housing 19 of the inhaler 1 and/or by a portion 30 of the cover 15. The portion 29 serves in particular for holding or gripping the inhaler 1 or as an abutment surface for a finger, especially a thumb, of the user (not shown). The portion 30 serves in particular as an actuating surface, preferably for a finger or thumb of the user (not shown), during opening or for the purpose of opening the cover 15. When the inhaler 1 is closed, the two portions 29 and 30 are preferably located side by side and form, in particular, an at least substantially continuous outer surface of the inhaler 1. To open the cover 15 the two portions 29 and 30 can preferably be pushed apart. This allows very simple and/or intuitive operation.

Generally it should be pointed out regarding the two embodiments that the carrier 3 is preferably moved or displaced at right angles or perpendicular to the direction of conveying or in the radial direction, in order to fluidically connect the respective receptacle or blister pouch 4 to the removal device 6 or the removal element 16 thereof, and/or to open said receptacle or pouch 4. The removal device 6 or its removal element 16 is correspondingly preferably fixed in construction. After the removal or inhalation of the formulation 2 from the respective blister pouch 4, the carrier is preferably moved back again in the opposite direction to allow the carrier to be conveyed onwards.

The lateral movement of the carrier for the purpose of opening it is preferably carried out by the opening or closing movement of the cover 15, particularly separately from the gear, particularly preferably by means of a sliding guide or other forced guide, which correspondingly moves or displaces the guide element 13 and hence the carrier or the receptacle located in the removal position, with the desired dependency on the movement of the cover 15 (in this case towards or away from the removal element 16)

Individual features and aspects of the two embodiments may also be combined with one another as desired.

Some preferred ingredients and/or compositions of the preferably medicinal formulation 2 are listed below. Particularly preferably the formulation 2 contains the following:
The compounds listed below may be used in the device according to the invention on their own or in combination. In the compounds mentioned below, **W** is a pharmacologically active substance and is selected (for example) from among the betamimetics, anticholinergics, corticosteroids, PDE4-inhibitors, LTD4-antagonists, EGFR-inhibitors, dopamine agonists, H1-antihistamines, PAF-antagonists and P13-kinase inhibitors. Moreover, double or triple combinations of **W** may be combined and used in the device according to the invention. Combinations of **W** might be, for example:
- **W** denotes a betamimetic, combined with an anticholinergic, corticosteroid, PDE4-inhibitor, EGFR-inhibitor or LTD4-antagonist,
- **W** denotes an anticholinergic, combined with a betamimetic, corticosteroid, PDE4-inhibitor, EGFR-inhibitor or LTD4-antagonist,
- **W** denotes a corticosteroid, combined with a PDE4-inhibitor, EGFR-inhibitor or LTD4-antagonist
- **W** denotes a PDE4-inhibitor, combined with an EGFR-inhibitor or LTD4-antagonist
- **W** denotes an EGFR-inhibitor, combined with an LTD4-antagonist.

The compounds used as betamimetics are preferably compounds selected from among albuterol, arformoterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmefamol, salmeterol, soterenol, sulphonterol, terbutaline, tiaramide, tolubuterol, zinterol, CHF-1035, HOKU-81, KUL-1248 and
- 3-(4-{6-[2-hydroxy-2-(4-hydroxy-3-hydroxymethyl-phenyl)-ethylamino]-hexyloxy}-butyl)-benzyl-sulphonamide
- 5-[2-(5.6-diethyl-indan-2-ylamino)-1-hydroxy-ethyl]-8-hydroxy-1H-quinolin-2-one
- 4-hydroxy-7-[2-{[2-{[3-(2-phenylethoxy)propyl]sulphonyl}ethyl]-amino}ethyl]-2(3H)-benzothiazolone
- 1-(2-fluoro-4-hydroxyphenyl)-2-[4-(1-benzimidazolyl)-2-methyl-2-butylamino]ethanol
- 1-[3-(4-methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-hydroxy-8-(1-hydroxy-2-isopropylaminobutyl)-2H-1,4-benzoxazin-3-(4H)-one
- 1-(4-amino-3-chloro-5-trifluoromethylphenyl)-2-tert.-butylamino)ethanol
- 6-hydroxy-8-{1-hydroxy-2-[2-(4-methoxy-phenyl)-1,1-dimethyl-ethylamino]-ethyl}-4H-benzo[1,4]oxazin-3-one
- 6-hydroxy-8-{1-hydroxy-2-[2-(ethyl 4-phenoxy-acetate)-1,1-dimethyl-ethylamino]-ethyl}-4H-benzo[1,4]oxazin-3-one
- 6-hydroxy-8-{1-hydroxy-2-[2-(4-phenoxy-acetic acid)-1,1-dimethyl-ethylamino]-ethyl}-4H-benzo[1,4]oxazin-3-one
- 8-{2-[1,1-dimethyl-2-(2.4.6-trimethylphenyl)-ethylamino]-1-hydroxy-ethyl}-6-hydroxy-4H-benzo[1,4]oxazin-3-one
- 6-hydroxy-8-{1-hydroxy-2-[2-(4-hydroxy-phenyl)-1,1-dimethyl-ethylamino]-ethyl}-4H-benzo[1,4]oxazin-3-one
- 6-hydroxy-8-{1-hydroxy-2-[2-(1.1dimethyl-ethylamino]-ethyl}-4H-benzo[1,4] oxazin-3-one
- 8-{2-[2-(4-ethyl-phenyl)-1,1-dimethyl-ethylamino]-1-hydroxy-ethyl}-6-hydroxy-4H-benzo[1,4]oxazin-3-one
- 8-{2-[2-(4-ethoxy-phenyl)-1,1-dimethyl-ethylamino]-1-hydroxy-ethyl}-6-hydroxy-4H-benzo[1,4]oxazin-3-one
- 4-(4-{2-[2-hydroxy-2-(6-hydroxy-3-oxo-3.4-dihydro-2H-benzo[1,4]oxazin-8-yl)-ethylamino]-2-methyl-propyl}-phenoxy)-butyric acid
- 8-{2-[2-(3.4-difluoro-phenyl)-1,1-dimethyl-ethylamino]-1-hydroxy-ethyl}-6-hydroxy-4H-benzo[1,4] oxazin-3 -one
- 1-(4-ethoxy-carbonylamino-3-cyano-5-fluorophenyl)-2-(tert-butylamino)ethanol
- 2-hydroxy-5-(1-hydroxy-2-{2-[4-(2-hydroxy-2-phenyl-ethylamino)-phenyl]-ethylamino}-ethyl)-benzaldehyde
- N-[2-hydroxy-5-(1-hydroxy-2-{2-[4-(2-hydroxy-2-phenyl-ethylamino)-phenyl]-ethylamino}-ethyl)-phenyl]-formamide
- 8-hydroxy-5-(1-hydroxy-2-{2-[4-(6-methoxy-biphenyl-3-ylamino)-phenyl]-ethylamino}-ethyl)-1H-quinolin-2-one
- 8-hydroxy-5-[1-hydroxy-2-(6-phenethylamino-hexylamino)-ethyl]-1H-quinolin-2-one
- 5-[2-(2-{4-[4-(2-amino-2-methyl-propoxy)-phenylamino]-phenyl}-ethylamino)-1-hydroxy-ethyl]-8-hydroxy-1H-quinolin-2-one
- [3-(4-{6-[2-hydroxy-2-(4-hydroxy-3-hydroxymethyl-phenyl)-ethylamino]-hexyloxy}-butyl)-5-methyl-phenyl]-urea
- 4-(2-{6-[2-(2.6-dichloro-benzyloxy)-ethoxy]-hexylamino}-1-hydroxy-ethyl)-2-hydroxymethyl-phenol
- 3-(4-{6-[2-hydroxy-2-(4-hydroxy-3-hydroxymethyl-phenyl)-ethylamino]-hexyloxy} -butyl)-benzylsulphonamide
- 3-(3-{7-[2-hydroxy-2-(4-hydroxy-3-hydroxymethyl-phenyl)-ethylamino]-heptyloxy}-propyl)-benzylsulphonamide
- 4-(2-{6-[4-(3-cyclopentanesulphonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamide
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The anticholinergics used are preferably compounds selected from among the tiotropium salts, preferably the bromide salt, oxitropium salts, preferably the bromide salt, flutropium salts, preferably the bromide salt, ipratropium salts, preferably the bromide salt, glycopyrronium salts, preferably the bromide salt, trospium salts, preferably the chloride salt, tolterodine. In the above-mentioned salts the cations are the pharmacologically active constituents. As anions the above-mentioned salts may preferably contain the chloride, bromide, iodide, sulphate, phosphate, methanesulphonate, nitrate, maleate, acetate, citrate, fumarate, tartrate, oxalate, succinate, benzoate or p-toluenesulphonate, while chloride, bromide, iodide, sulphate, methanesulphonate or p-toluenesulphonate are preferred as counter-ions. Of all the salts the chlorides, bromides, iodides and methanesulphonates are particularly preferred.

Other preferred anticholinergics are selected from among the salts of formula **AC-1** 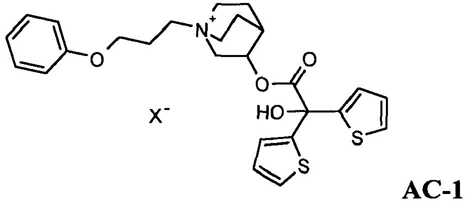 wherein X⁻ denotes an anion with a single negative charge, preferably an anion selected from among the fluoride, chloride, bromide, iodide, sulphate, phosphate, methanesulphonate, nitrate, maleate, acetate, citrate, fumarate, tartrate, oxalate, succinate, benzoate and p-toluenesulphonate, preferably an anion with a single negative charge, particularly preferably an anion selected from among the fluoride, chloride, bromide, methanesulphonate and p-toluenesulphonate, particularly preferably bromide, optionally in the form of the racemates, enantiomers or hydrates thereof. Of particular importance are those pharmaceutical combinations which contain the enantiomers of formula **AC-1-en** 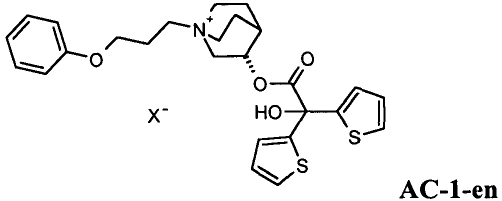 wherein X⁻ may have the above-mentioned meanings. Other preferred anticholinergics are selected from the salts of formula **AC-2** 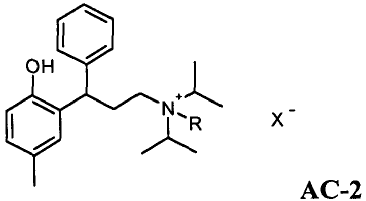 wherein R denotes either methyl or ethyl and wherein X⁻ may have the above-mentioned meanings. In an alternativen embodiment the compound of formula AC-2 may also be present in the form of the free base **AC-2-base.**

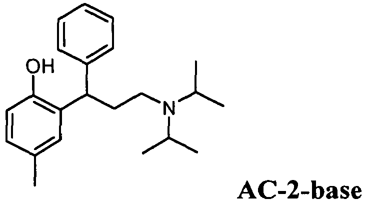

Other specified compounds are:
- tropenol 2,2-diphenylpropionate methobromide,
- scopine 2,2-diphenylpropionate methobromide,
- scopine 2-fluoro-2,2-diphenylacetate methobromide,
- tropenol 2-fluoro-2,2-diphenylacetate methobromide;
- tropenol 3,3',4,4'-tetrafluorobenzilate methobromide,
- scopine 3,3',4,4'-tetrafluorobenzilate methobromide,
- tropenol 4,4'-difluorobenzilate methobromide,
- scopine 4,4'-difluorobenzilate methobromide,
- tropenol 3,3'-difluorobenzilate methobromide,
- scopine 3,3'- difluorobenzilate methobromide;
- tropenol 9-hydroxy-fluorene-9-carboxylate methobromide;
- tropenol 9-fluoro-fluorene-9-carboxylate methobromide;
- scopine 9-hydroxy-fluorene-9- carboxylate methobromide;
- scopine 9-fluoro-fluorene-9- carboxylate methobromide;
- tropenol 9-methyl-fluorene-9- carboxylate methobromide;
- scopine 9-methyl-fluorene-9- carboxylate methobromide;
- cyclopropyltropine benzilate methobromide;
- cyclopropyltropine 2,2-diphenylpropionate methobromide;
- cyclopropyltropine 9-hydroxy-xanthene-9-carboxylate methobromide;
- cyclopropyltropine 9-methyl-fluorene-9-carboxylate methobromide;
- cyclopropyltropine 9-methyl-xanthene-9-carboxylate methobromide;
- cyclopropyltropine 9-hydroxy-fluorene-9-carboxylate methobromide;
- cyclopropyltropine methyl 4,4'-difluorobenzilate methobromide.
- tropenol 9-hydroxy-xanthene-9-carboxylate methobromide;
- scopine 9-hydroxy-xanthene-9-carboxylate methobromide;
- tropenol 9-methyl-xanthene-9-carboxylate -methobromide;
- scopine 9-methyl-xanthene-9-carboxylate -methobromide;
- tropenol 9-ethyl-xanthene-9-carboxylate methobromide;
- tropenol 9-difluoromethyl-xanthene-9-carboxylate methobromide;
- scopine 9-hydroxymethyl-xanthene-9-carboxylate methobromide,

The above-mentioned compounds may also be used as salts within the scope of the present invention, wherein instead of the methobromide the salts metho-X are used, wherein X may have the meanings given hereinbefore for X⁻.

As corticosteroids it is preferable to use compounds selected from among beclomethasone, betamethasone, budesonide, butixocort, ciclesonide, deflazacort, dexamethasone, etiprednol, flunisolide, fluticasone, loteprednol, mometasone, prednisolone, prednisone, rofleponide, triamcinolone, RPR-106541, NS-126, ST-26 and
- (S)-fluoromethyl 6,9-difluoro-17-[(2-furanylcarbonyl)oxy]-11-hydroxy-16-methyl-3-oxo-androsta-1,4-diene-17-carbothionate
- (S)-(2-oxo-tetrahydro-furan-3S-yl)6,9-difluoro-11-hydroxy-16-methy1-3-oxo-17-propionyloxy-androsta-1,4-diene-17-carbothionate,
- cyanomethyl 6α,9α-difluoro-11β-hydroxy-16α-methyl-3-oxo-17α-(2,2,3,3-tertamethylcyclopropylcarbonyl)oxy-androsta-1,4-diene-17β-carboxylate
optionally in the form of the racemates, enantiomers or diastereomers thereof and optionally in the form of the salts and derivatives thereof, the solvates and/or hydrates thereof. Any reference to steroids includes a reference to any salts or derivatives, hydrates or solvates thereof which may exist. Examples of possible salts and derivatives of the steroids may be: alkali metal salts, such as for example sodium or potassium salts, sulphobenzoates, phosphates, isonicotinates, acetates, dichloroacetates, propionates, dihydrogen phosphates, palmitates, pivalates or furoates.

PDE4-inhibitors which may be used are preferably compounds selected from among enprofyllin, theophyllin, roflumilast, ariflo (cilomilast), tofimilast, pumafentrin, lirimilast, arofyllin, atizoram, D-4418, Bay-198004, BY343, CP-325.366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 and
- N-(3,5-dichloro-1-oxo-pyridin-4-yl)-4-difluoromethoxy-3-cyclopropy lmethoxybenzamide
- (-)p-[(4*a*R*,10*b*S*)-9-ethoxy-1,2,3,4,4a,10b-hexahydro-8-methoxy-2-methylbenzo[s][1,6]naphthyridin-6-yl]-N,N-diisopropylbenzamide
- (R)-(+)-1-(4-bromobenzyl)-4-[(3-cyclopentyloxy)-4-methoxyphenyl]-2-pyrrolidone
- 3-(cyclopentyloxy-4-methoxyphenyl)-1-(4-N'-[N-2-cyano-S-methyl-isothioureido]benzyl)-2-pyrrolidone
- cis[4-cyano-4-(3-cyclopentyloxy-4-methoxyphenyl)cyclohexane-1-carboxylic acid]
- 2-carbomethoxy-4-cyano-4-(3-cyclopropylmethoxy-4-difluoromethoxyphenyl)cyclohexan-1-one
- cis[4-cyano-4-(3-cyclopropylmethoxy-4-difluoromethoxyphenyl)cyclohexan-1-ol]
- (R)-(+)-ethyl[4-(3-cyclopentyloxy-4-methoxyphenyl)pyrrolidin-2-ylidene]acetate
- (S)-(-)-ethyl[4-(3-cyclopentyloxy-4-methoxyphenyl)pyrrolidin-2-ylidene]acetate
- 9-cyclopentyl-5,6-dihydro-7-ethyl-3-(2-thieny1)-9*H*-pyrazolo[3.4-c]-1,2,4-triazolo[4.3-a]pyridine
- 9-cyclopentyl-5,6-dihydro-7-ethyl-3-(*tert*-buty1)-9*H*-pyrazolo[3.4-c]-1,2,4-triazolo[4.3-a]pyridine
optionally in the form of the racemates, enantiomers or diastereomers thereof and optionally in the form of the pharmacologically acceptable acid addition salts thereof, the solvates and/or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The LTD4-antagonists used are preferably compounds selected from among montelukast, pranlukast, zafirlukast, MCC-847 (ZD-3523), MN-001, MEN-91507 (LM-1507), VUF-5078, VUF-K-8707, L-733321 and
- 1-(((R)-(3-(2-(6,7-difluoro-2-quinolinyl)ethenyl)phenyl)-3-(2-(2-hydroxy-2-propyl)phenyl)thio)methylcyclopropane-acetic acid,
- 1-(((1(R)-3(3-(2-(2,3-dichlorothieno[3,2-b]pyridin-5-yI)-(E)-ethenyl)phenyl)-3-(2-(1-hydroxy-1-methylethyl)phenyl)propyl)thio)methyl)cyclopropane-acetic acid
- [2-[[2-(4-tert-butyl-2-thiazolyl)-5-benzofuranyl]oxymethyl]phenyl]acetic acid
optionally in the form of the racemates, enantiomers or diastereomers thereof and optionally in the form of the pharmacologically acceptable acid addition salts, solvates and/or hydrates thereof. According to the invention the acid addition salts of the betamimetics are preferably selected from among the hydrochloride, hydrobromide, hydroiodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate. By salts or derivatives which the LTD4-antagonists may optionally be capable of forming are meant, for example: alkali metal salts, such as for example sodium or potassium salts, alkaline earth metal salts, sulphobenzoates, phosphates, isonicotinates, acetates, propionates, dihydrogen phosphates, palmitates, pivalates or furoates.

EGFR-inhibitors which may be used are preferably compounds selected from among cetuximab, trastuzumab, ABX-EGF, Mab ICR-62 and
- 4-[(3-chloro-4-fluorophenyl)amino]-6-{[4-(morpholin-4-yl)-1-oxo-2-buten-1-yl]amino}-7-cyclopropylmethoxy-quinazoline
- 4-[(3-chloro-4-fluorophenyl)amino]-6-{[4-(N,N-diethylamino)-1-oxo-2-buten-1-y1]amino}-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopropylmethoxy-quinazoline
- 4-[(R)-(1-phenyl-ethyl)amino]-6-{[4-(morpholin-4-yl)-1-oxo-2-buten-1-yl]-amino}-7-cyclopentyloxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline
- 4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline
- 4-[(3-chloro-4-fluoro-phenyl)amino]-6-[2-((S)-6-methyl-2-oxo-morpholin-4-yl)-ethoxy]-7-methoxy-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopentyloxy-quinazoline
- 4-[(R)-(1-phenyl-ethyl)amino]-6-{[4-(N,N-to-(2-methoxy-ethyl)-amino)-1-oxo-2-buten-1-yl]amino}-7-cyclopropylmethoxy-quinazoline
- 4-[(R)-(1-pheny1-ethyl)amino]-6-({4-[N-(2-ntethoxy-ethyl)-N-ethyl-amino]-1-oxo-2-buten-1-yl}amino)-7-cyclopropylmethoxy-quinazoline
- 4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline
- 4-[(R)-(1-phenyl-ethyl)amino]-6-({4-[N-(tetrahydropyran-4-yl)-N-methyl-amino]-1-oxo-2-buten-1-y1}amino)-7-cyclopropylmethoxy-quinazoline
- 4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline
- 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopentyloxy-quinazoline
- 4-[(3-chloro-4-fluorophenyl)amino]-6-{[4-(N-cyclopropyl-N-methyl-amino)-1-oxo-2-buten-1-yl]amino} -7-cyclopentyloxy-quinazoline
- 4-[(3-chloro-4-fluorophenyl)amino]-6- {[4-(N,N-dimethylamino)-1-oxo-2-buten-1-yl]amino}-7-[(R)-(tetrahydrofuran-2-yl)methoxy]-quinazoline
- 4-[(3-chloro-4-fluorophenyl)amino]-6-{[4-(N,N-dimethylamino)-1-oxo-2-buten-1-yl]amino)-7-[(S)-(tetrahydrofuran-2-yl)methoxy]-quinazoline
- 4-[(3-ethynyl-phenyl)amino]-6.7-to-(2-methoxy-ethoxy)-quinazoline
- 4-[(3-chloro-4-fluorophenyl)amino]-7-[3-(morpholin-4-yl)-propyloxy]-6-[(vinylcarbonyl)amino]-quinazoline
- 4-[(R)-(1-phenyl-ethyl)amino]-6-(4-hydroxy-phenyl)-7H-pyrrolo[2,3-d]pyrimidine
- 3-cyano-4-[(3-chloro-4-fluorophenyl)amino]-6-{[4-(N,N-dimethylamino)-1-oxo-2-buten-1-yl]amino}-7-ethoxy-quinoline
- 4-{[3-chloro-4-(3-fluoro-benzyloxy)-phenyl]amino}-6-(5-{[(2-methanesulphonyl-ethyl)amino]methyl}-furan-2-yl)quinazoline
- 4-[(R)-(1-phenyl-ethyl)amino]-6-{[4-((R)-6-methyl-2-oxo-morpholin-4-yl)-1-oxo-2-buten-1-yl]amino}-7-methoxy-quinazoline
- 4-[(3-chloro-4-fluorophenyl)amino]-6-{[4-(morpholin-4-yl)-1-oxo-2-buten-1-yl]amino}-7-[(tetrahydrofuran-2-yl)methoxy]-quinazoline
- 4-[(3-chloro-4-fluorophenyl)amino]-6-({4-[N,N-to-(2-methoxy-ethyl)-amino]-1-oxo-2-buten-1-yl}amino)-7-[(tetrahydrofuran-2-yl)methoxy]-quinazoline
- 4-[(3-ethynyl-phenyl)amino]-6-{ [4-(5.5-dimethyl-2-oxo-morpholin-4-yl)-1-oxo-2-buten-1-yl]amino}-quinazoline
- 4-[(3-chloro-4-fluoro-phenyl)amino]-6-[2-(2,2-dimethyl-6-oxo-morpholin-4-yl)-ethoxy]-7-methoxy-quinazoline
- 4-[(3-chloro-4-fluoro-phenyl)amino]-6-[2-(2,2-dimethyl-6-oxo-morpholin-4-yl)-ethoxy]-7-[(R)-(tetrahydrofuran-2-yl)methoxy]-quinazoline
- 4-[(3-chloro-4-fluoro-phenyl)amino]-7-[2-(2,2-dimethyl-6-oxo-morpholin-4-yl)-ethoxy]-6-[(S)-(tetrahydrofuran-2-yl)methoxy]-quinazoline
- 4-[(3-chloro-4-fluoro-phenyl)amino]-6-{2-[4-(2-oxo-morpholin-4-yl)-piperidin-1-yl]-ethoxy}-7-methoxy-quinazoline
- 4-[(3-chloro-4-fluoro-phenyl)amino]-6-[1-(tert.-butyloxycarbonyl)-piperidin-4-yloxy]-7-methoxy-quinazoline
- 4-[(3-chloro-4-fluoro-phenyl)amino]-6-(trans-4-amino-cyclohexan-1-yloxy)-7-methoxy-quinazoline
- 4-[(3-chloro-4-fluoro-phenyl)amino]-6-(trans-4-methanesulphonylamino-cyclohexan-1-yloxy)-7-methoxy-quinazoline
- 4-[(3-chloro-4-fluoro-phenyl)amino]-6-(tetrahydropyran-3-yloxy)-7-methoxy-quinazoline
- 4-[(3-chloro-4-fluoro-phenyl)amino]-6-(1-methyl-piperidin-4-yloxy)-7-methoxy-quinazoline
- 4-[(3-chloro-4-fluoro-phenyl)amino]-6-{1-[(morpholin-4-yl)carbonyl]-piperidin-4-yloxy }-7-methoxy-quinazoline
- 4-[(3-chloro-4-fluoro-phenyl)amino]-6-{1-[(methoxymethyl)carbonyl]-piperidin-4-yloxy}-7-methoxy-quinazoline
- 4-[(3-chloro-4-fluoro-phenyl)amino]-6-(piperidin-3-yloxy)-7-methoxyquinazoline
- 4-[(3-chloro-4-fluoro-phenyl)amino]-6-[1-(2-acetylamino-ethyl)-piperidin-4-yloxy]-7-methoxy-quinazoline
- 4-[(3-chloro-4-fluoro-phenyl)amino]-6-(tetrahydropyran-4-yloxy)-7-ethoxy-quinazoline
- 4-[(3-chloro-4-fluoro-phenyl)amino]-6-((S)-tetrahydrofuran-3-yloxy)-7-hydroxy-quinazoline
- 4-[(3-chloro-4-fluoro-phenyl)amino]-6-(tetrahydropyran-4-yloxy)-7-(2-methoxy-ethoxy)-quinazoline
- 4-[(3-chloro-4-fluoro-phenyl)amino]-6-{trans-4-[(dimethylamino)sulphonylamino]-cyclohexan-1-yloxy)-7-methoxy-quinazoline
- 4-[(3-chloro-4-fluoro-phenyl)amino]-6-{trans-4-[(morpholin-4-yl)carbonylaminol-cyclohexan-1-yloxy)-7-methoxy-quinazoline
- 4-[(3-chloro-4-fluoro-phenyl)amino]-6- {trans-4-[(morpholin-4-yl)sulphonylamino]-cyclohexan-1 -yloxy}-7-methoxy-quinazoline
- 4-[(3-chloro-4-fluoro-phenyl)amino]-6-(tetrahydropyran-4-yloxy)-7-(2-acetylamino-ethoxy)-quinazoline
- 4-[(3-chloro-4-fluoro-phenyl)amino]-6-(tetrahydropyran-4-yloxy)-7-(2-methanesulphonylamino-ethoxy)-quinazoline
- 4-[(3-chloro-4-fluoro-phenyl)amino]-6-{1-[(piperidin-1-yl)carbonyl]-piperidin-4-yloxy}-7-methoxy-quinazoline
- 4-[(3-chloro-4-fluoro-phenyl)amino]-6-(1-aminocarbonylmethyl-piperidin-4-yloxy)-7-methoxy-quinazoline
- 4-[(3-chloro-4-fluoro-phenyl)amino]-6-(cis-4-{N-[(tetrahydropyran-4-yl)carbonyl]-N-methyl-amino }-cyclohexan-1-yloxy)-7-methoxy-quinazoline
- 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline
- 4-[(3-chloro-4-fluoro-phenyl)amino]-6-(cis-4- {N-[(morpholin-4-yl)sulphonyl]-N-methyl-amino}-cyclohexan-1-yloxy)-7-methoxy- quinazoline
- 4-[(3-chloro-4-fluoro-phenyl)amino]-6-(trans-4-ethanesulphonylamino-cyclohexan-1-yloxy)-7-methoxy-quinazoline
- 4- [(3 -chloro-4-fluoro-phenyl)amino]-6-(1 -methanesulphonyl-piperidin-4-yloxy)-7-ethoxy-quinazoline
- 4-[(3-chloro-4-fluoro-phenyl)amino]-6-(1-methanesulphonyl-piperidin-4-yloxy)-7-(2-methoxy-ethoxy)-quinazoline
- 4-[(3-chloro-4-fluoro-phenyl)ammo]-6-[1-(2-methoxy-acctyl)-piperidin-4-yloxy]-7-(2-methoxy-ethoxy)-quinazoline
- 4-[(3-chloro-4-fluoro-phenyl)amino]-6-(cis-4-acetylamino-cyclohexan-1-yloxy)-7-methoxy-quinazoline
- 4-[(3-ethynyl-phenyl)amino]-6-[1-(tert.-butyloxycarbonyl)-piperidin-4-yloxy]-7-methoxy-quinazoline
- 4-[(3-ethynyl-phenyl)amino]-6-(tetrahydropyran-4-yloxy]-7-methoxyquinazoline
- 4-[(3-chloro-4-fluoro-phenyl)amino]-6-(cis-4- {N-[(piperidin-1-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(4-methyl-piperazin-1-yl)carbonyl]-N-methyl-amino}-cyclohexan-1-yloxy)-7-methoxy-quinazoline
- 4-[(3-chloro-4-fluoro-phenyl)amino]-6-{cis-4-[(morpholin-4-yl)carbonylamino]-cyclohexan-1-yloxy}-7-methoxy-quinazoline
- 4-[(3-chloro-4-fluoro-phenyl)amino]-6-{1-[2-(2-oxopyrrolidin-1-yl)ethyl]-piperidin-4-yloxy}-7-methoxy-quinazoline
- 4-[(3-chloro-4-fluoro-phenyl)amino]-6-{1-[(morpholin-4-yl)carbonyl]-piperidin-4-yloxy}-7-(2-methoxy-ethoxy)-quinazoline
- 4-[(3-ethynyl-phenyl)amino]-6-(1-acetyl-piperidin-4-yloxy)-7-methoxy-quinazoline
- 4-[(3-ethynyl-phenyl)amino]-6-(1-methyl-piperidin-4-yloxy)-7-methoxyquinazoline
- 4-[(3-ethynyl-phenyl)amino]-6-(1-methanesulphonyl-piperidin-4-yloxy)-7-methoxy-quinazoline
- 4-[(3-chloro-4-fluoro-phenyl)amino]-6-(1-methyl-piperidin-4-yloxy)-7(2-methoxy-ethoxy)-quinazoline
- 4-[(3-chloro-4-fluoro-phenyl)amino]-6-(1-isopropyloxycarbonyl-piperidin-4-yloxy)-7-methoxy-quinazoline
- 4-[(3-chloro-4-fluoro-phenyl)amino]-6-(cis-4-methylamino-cyclohexan-1-yloxy)-7-methoxy-quinazoline
- 4-[(3-chloro-4-fluoro-phenyl)amino]-6-{cis-4-[N-(2-methoxy-acetyl)-N-methyl-amino]-cyclohexan-1-yloxyl-7-methoxy-quinazoline
- 4-[(3-ethynyl-phenyl)amino]-6-(piperidin-4-yloxy)-7-methoxy-quinazoline
- 4-[(3-ethynyl-phenyl)amino]-6-[1-(2-methoxy-acetyl)-piperidin-4-yloxy]-7-methoxy-quinazoline
- 4-[(3-ethynyl-phenyl)amino]-6-{1-[(morpholin-4-yl)carbonyl]-piperidin-4-yloxy}-7-methoxy-quinazoline
- 4-[(3-chloro-4-fluoro-phenyl)amino]-6-{1-[(cis-2,6-dimethyl-morpholin-4-yl)carbonyl]-piperidin-4-yloxy}-7-methoxy-quinazoline
- 4- [(3-chloro-4-fluoro-phenyl)amino]-6-{1-[(2-methyl-morpholin-4-yl)carbonyl]-piperidin-4-yloxy}-7-methoxy-quinazoline
- 4-[(3-chloro-4-fluoro-phenyl)amino]-6-{1-[(S,S)-(2-oxa-5-aza-bicyclo[2,2,1]hept-5-yl)carbonyl]-piperidin-4-yloxy}-7-methoxy-quinazoline
- 4-[(3-chloro-4-fluoro-phenyl)amino]-6-{1-[(N-methyl-N-2-methoxyethyl-amino)carbonyl]-piperidin-4-yloxy} -7-methoxy-quinazoline
- 4-[(3-chloro-4-fluoro-phenyl)amino]-6-(1-ethyl-piperidin-4-yloxy)-7-methoxy-quinazoline
- 4-[(3-chloro-4-fluoro-phenyl)amino]-6-{1-[(2-methoxyethyl)carbonyl]-piperidin-4-yloxy}-7-methoxy-quinazotme
- 4-[(3-chloro-4-fluoro-phenyl)amino]-6-{1-[(3-methoxypropyl-amino)-carbonyl]-piperidin-4-yloxy}-7-methoxy-quinazoline
- 4-[(3-chloro-4-fluoro-phenyl)amino]-6-[cis-4-(N-methanesulphonyl-N-methyl-amino)-cyclohexan-1-yloxy]-7-methoxy-quinazoline
- 4-[(3-chloro-4-fluoro-phenyl)amino]-6-[cis-4-(N-acetyl-N-methyl-amino)-cyclohexan-1-yloxy]-7-methoxy-quinazoline
- 4-[(3-chloro-4-fluoro-phenyl)amino]-6-(trans-4-methylamino-cyclohexan-1-yloxy)-7-methoxy-quinazoline
- 4-[(3-chloro-4-fluoro-phenyl)amino]-6-[trans-4-(N-methanesulphonyl-N-methyl-amino)-cyclohexan-1-yloxy]-7-methoxy-quinazoline
- 4-[(3-chloro-4-fluoro-phenyl)amino]-6-(trans-4-dimethylamino-cyclohexan-1-yloxy)-7-methoxy-quinazoline
- 4-[(3-chloro-4-fluoro-phenyl)amino]-6-(trans-4-{N-[(morpholin-4-yl)carbonyl]-N-methyl-amino}-cyclohexan-1-yloxy)-7-methoxy-quinazoline
- 4-[(3-chloro-4-fluoro-phenyl)amino]-6-[2-(2,2-dimethyl-6-oxo-morpholin-4-yl)-ethoxy]-7-[(S)-(tetrahydrofuran-2-yl)methoxy]-quinazoline
- 4-[(3-chloro-4-fluoro-phenyl)amino]-6-(1-methanesulphonyl-piperidin-4-yloxy)-7-methoxy-quinazoline
- 4-[(3-chloro-4-fluoro-phenyl)amino]-6-(1-cyano-piperidin-4-yloxy)-7-methoxy-quinazoline
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The dopamine agonists used are preferably compounds selected from among bromocriptin, cabergoline, alpha-dihydroergocryptine, lisuride, pergolide, pramipexol, roxindol, ropinirol, talipexol, tergurid and viozan, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydrooxalate, hydrosuccinate, hydrobenzoate and hydro-p-toluenesulphonate.

HI-Antihistamines which may be used are preferably compounds selected from among epinastine, cetirizine, azelastine, fexofenadine, levocabastine, loratadine, mizolastine, ketotifen, emedastine, dimetindene, clemastine, bamipine, cexchlorpheniramine, pheniramine, doxylamine, chlorophenoxamine, dimenhydrinate, diphenhydramine, promethazine, ebastine, desloratidine and meclozine, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

It is also possible to use inhalable macromolecules, as disclosed in EP 1 003 478 A1 or CA 2297174 A1.

In addition, the compounds may come from the groups of ergot alkaloid derivatives, the triptans, the CGRP-inhibitors, the phosphodiesterase-V inhibitors, optionally in the form of the racemates, enantiomers or diastereomers thereof, optionally in the form of the pharmacologically acceptable acid addition salts, the solvates and/or hydrates thereof.

Examples of ergot alkaloid derivatives are dihydroergotamine and ergotamine.

## Claims

1. Inhaler (1) for delivering a powdered formulation (2) from a carrier which is band-shaped, flexible and forming a closed loop, with a plurality of receptacles, each of which contains one dose of the formulation (2),
with a conveying device (5) for stepwise advancing of the carrier, which comprises in particular two deflectors (8) between which the carrier extends,
wherein the carrier extends over a circumferential angle (α) of the inhaler (1) of less than 360°,
with a removal device (6) for separately opening the receptacles, wherein the removal device (6) comprises a removal element (16),
**characterized in that**
the removal device (6) comprises a guide element (13) which is movable for individually opening the receptacles by moving the respective receptacle or the carrier relative to the removal element (16).

2. Inhaler according to claim 1, wherein the carrier is guided between the two deflectors (8) in each case with an at least substantially constant curvature.

3. Inhaler according to claim 1 or 2, **characterised in that** the carrier extends exclusively between the two deflectors (8), and/or that the carrier is guided between the two deflectors (8), at least substantially along an outer and inner arc, particularly an arc of a circle.

4. Inhaler according to one of the preceding claims, **characterized in that** the deflectors (8) deflect the carrier through at least 160° each, particularly at least substantially 180° or more each.

5. Inhaler according to one of the preceding claims, **characterized in that** the portions of the carrier between the two deflectors (8) are guided at an at least substantially constant spacing from one another.

6. Inhaler (1) according to one of the preceding claims,
with a conveying device (5) which comprises a plurality of wheels (12) for stepwise advancing and/or for guiding the carrier,
wherein the wheels (12) have the same diameter, are arranged on a common radius, can be driven by common drive means, particularly a sun wheel (14), and/or have the same direction of rotation.

7. Inhaler according to claim 6, **characterised in that** the diameter of the wheels (12) essentially corresponds to the spacing of the portions, wherein the portions of the carrier between the two deflectors (8) are guided at an at least substantially constant spacing from one another.

8. Inhaler (1) according to one of the preceding claims,
with a conveying device (5) having a gear, particularly a planet gear (21), for advancing the carrier, the conveying device (5) having a free wheel clutch (22), preferably wherein the free wheel clutch (22) is arranged in a drive wheel and/or gear wheel, particularly in a sun wheel (14), and/or preferably wherein the conveying device (5) has a non-return mechanism (28) which forms a travel limiter for the reverse movement of the carrier, particularly in order to be able to position the respective receptacle in a defined position for the next delivery.

9. Inhaler according to one of the preceding claims, **characterised in that** the conveying device (5) has in particular only two or four wheels (12) which act on the carrier, and/or that all the wheels (12) acting on the carrier are driven, and/or that the conveying device (5) forms the sole drive for moving the carrier.

10. Inhaler according to one of the preceding claims, **characterised in that** the inhaler (1) has a mouthpiece (7) and an associated cover (15) which is movable or pivotable for opening and/or closing the mouthpiece (7), wherein the inhaler (1) is constructed such that the conveyor device (5) and/or the removal device (6) can be actuated or driven in order to open the individual receptacles and/or remove the doses of formulation (2) by means of the opening and/or closing movement of the cover (15).

11. Inhaler according to one of the preceding claims, **characterised in that** the guide element (13) is movable in the manner of a sled and/or at right angles to the direction of conveying or advancing of the carrier.

12. Inhaler according to one of the preceding claims, **characterised in that** the removal element (16) is constructed as a piercing element, fixedly formed and/or attached to a mouthpiece (7) of the inhaler (1).

13. Inhaler (1) according to one of the preceding claims, wherein the removal element (16) is at least substantially stationary, and wherein
the carrier is movable at right angles to the direction of advance towards the removal element (16) in order to open the carrier or a receptacle of the carrier or to connect to the removal element (16).

14. Inhaler (1) according to one of the preceding claims,
with a substantially round housing (19) which is flattened or indented on one side, preferably wherein the flattened or indented side is formed by a portion (29) of the housing (19) and by a portion (30) of a cover (15) of the inhaler (1) which is adjacent to said portion (29) when the inhaler (1) is closed.

15. Inhaler according to one of the preceding claims, **characterised in that** the inhaler (1) is portable in construction.

## Patentansprüche

1. Inhalator (1) zur Ausgabe einer pulverförmigen Formulierung (2) aus einem bandförmigen, flexiblen und eine geschlossene Schleife bildenden Träger, mit einer Vielzahl von Aufnahmen, die jeweils eine Dosis der Formulierung (2) enthalten,
mit einer Fördervorrichtung (5) zum schrittweisen Vorwärtsbewegen des Trägers, die insbesondere zwei Umlenkungen (8) aufweist, zwischen denen sich der Träger erstreckt,
wobei sich der Träger über einen Umfangswinkel (α) des Inhalators (1) von weniger als 360° erstreckt,
mit einer Entnahmevorrichtung (6) zum separaten Öffnen der Aufnahmen, wobei die Entnahmevorrichtung (6) ein Entnahmeelement (16) umfasst,
**dadurch gekennzeichnet, dass**
die Entnahmevorrichtung (6) ein Führungselement (13) umfasst, das zum einzelnen Öffnen der Aufnahmen bewegt werden kann, indem die jeweilige Aufnahme oder der Träger relativ zu dem Entnahmeelement (16) bewegt wird.

2. Inhalator nach Anspruch 1, wobei der Träger zwischen den zwei Umlenkungen (8) in jedem Fall mit einer wenigstens im Wesentlichen konstanten Krümmung geführt wird.

3. Inhalator nach Anspruch 1 oder 2, **dadurch gekennzeichnet, dass** sich der Träger ausschließlich zwischen den zwei Umlenkungen (8) erstreckt und/oder dass der Träger zwischen den zwei Umlenkungen (8) wenigstens im Wesentlichen entlang eines äußeren und inneren Bogens, insbesondere eines Kreisbogens, geführt wird.

4. Inhalator nach einem der vorhergehenden Ansprüche, **dadurch gekennzeichnet, dass** die Umlenkungen (8) den Träger jeweils um wenigstens 160°, insbesondere wenigsten im Wesentlichen um 180° oder mehr umlenken.

5. Inhalator nach einem der vorhergehenden Ansprüche, **dadurch gekennzeichnet, dass** die Abschnitte des Trägers zwischen den zwei Umlenkungen (8) mit wenigstens im Wesentlichen konstantem Abstand voneinander geführt werden.

6. Inhalator (1) nach einem der vorhergehenden Ansprüche,
mit einer eine Vielzahl von Räder (12) umfassenden Fördervorrichtung (5) zum schrittweisen Vorwärtsbewegen und/oder zum Führen des Trägers,
wobei die Räder (12) den gleichen Durchmesser aufweisen, an einem gemeinsamen Radius angeordnet sind, durch gemeinsame Antriebsmittel, insbesondere ein Sonnenrad (14), angetrieben werden können und/oder die gleiche Drehrichtung aufweisen.

7. Inhalator nach Anspruch 6, **dadurch gekennzeichnet, dass** der Durchmesser der Räder (12) weitgehend dem Abstand der Abschnitte entspricht, wobei die Abschnitte des Trägers zwischen den zwei Umlenkungen (8) mit wenigstens im Wesentlichen konstantem Abstand voneinander geführt werden.

8. Inhalator (1) nach einem der vorhergehenden Ansprüche,
mit einer ein Getriebe, insbesondere ein Planetengetriebe (21), aufweisenden Fördervorrichtung (5) zum Vorwärtsbewegen des Trägers, wobei die Fördervorrichtung (5) eine Freilaufkupplung (22) aufweist, wobei die Freilaufkupplung (22) vorzugsweise in einem Antriebsrad und/oder Getrieberad, insbesondere in einem Sonnenrad (14), angeordnet ist und/oder wobei die Fördervorrichtung (5) vorzugsweise einen Rücklaufsicherungsmechanismus (28) aufweist, der einen Wegbegrenzer für die Rückwärtsbewegung des Trägers bildet, insbesondere um die jeweilige Aufnahme in einer definierten Position für die nächste Ausgabe positionieren zu können.

9. Inhalator nach einem der vorhergehenden Ansprüche, **dadurch gekennzeichnet, dass** die Fördervorrichtung (5) insbesondere nur zwei oder vier Räder (12) aufweist, die auf den Träger einwirken, und/oder dass alle auf den Träger einwirkenden Räder (12) angetrieben werden, und/oder dass die Fördervorrichtung (5) den einzigen Antrieb zum Bewegen des Trägers bildet.

10. Inhalator nach einem der vorhergehenden Ansprüche, **dadurch gekennzeichnet, dass** der Inhalator (1) ein Mundstück (7) und eine zugeordnete Abdeckung (15) aufweist, die zum Öffnen und/oder Schließen des Mundstücks (7) beweglich oder schwenkbar ist, wobei der Inhalator (1) derart ausgebildet ist, dass die Fördervorrichtung (5) und/oder die Entnahmevorrichtung (6) betätigt oder angetrieben werden können, um durch die Öffhungs- und/oder Schließbewegung der Abdeckung (15) die einzelnen Aufnahmen zu öffnen und/oder die Dosen der Formulierung (2) zu entnehmen.

11. Inhalator nach einem der vorhergehenden Ansprüche, **dadurch gekennzeichnet, dass** das Führungselement (13) in der Art eines Schlittens und/oder im rechten Winkel zur Förder- oder Vorwärtsbewegungsrichtung des Trägers beweglich ist.

12. Inhalator nach einem der vorhergehenden Ansprüche, **dadurch gekennzeichnet, dass** das Entnahmeelement (16) als Anstechelement ausgeführt, feststehend ausgebildet und/oder an einem Mundstück (7) des Inhalators (1) angebracht ist.

13. Inhalator (1) nach einem der vorhergehenden Ansprüche, wobei das Entnahmeelement (16) wenigstens im Wesentlichen stationär ist, und wobei
der Träger im rechten Winkel zur Vorschubrichtung in Richtung auf das Entnahmeelement (16) beweglich ist, um den Träger oder eine Aufnahme des Trägers zu öffnen oder mit dem Entnahmeelement (16) zu verbinden.

14. Inhalator (1) nach einem der vorhergehenden Ansprüche,
mit einem im Wesentlichen runden Gehäuse (19), das an einer Seite abgeflacht oder gekerbt ist, wobei die abgeflachte oder gekerbte Seite vorzugsweise durch einen Abschnitt (29) des Gehäuses (19) und einen Abschnitt (30) einer Abdeckung (15) des Inhalators (1) gebildet ist, der benachbart zu dem Abschnitt (29) ist, wenn der Inhalator (1) geschlossen ist.

15. Inhalator nach einem der vorhergehenden Ansprüche, **dadurch gekennzeichnet, dass** der Inhalator (1) tragbar ausgebildet ist.

## Revendications

1. Inhalateur (1) pour l'administration d'une formulation pulvérulente (2) depuis un support qui est en forme de bande, flexible et formant une boucle fermée, avec une pluralité de réceptacles, contenant chacun une dose de la formulation (2),
avec un dispositif d'acheminement (5) pour faire progresser le support pas à pas, qui comprend en particulier deux déflecteurs (8) entre lesquels s'étend le support,
dans lequel le support s'étend sur un angle circonférentiel (α) de l'inhalateur (1) inférieur à 360°,
avec un dispositif de retrait (6) pour séparément ouvrir les réceptacles, dans lequel le dispositif de retrait (6) comprend un élément de retrait (16),
**caractérisé en ce que**
le dispositif de retrait (6) comprend un élément de guidage (13) qui est mobile pour individuellement ouvrir les réceptacles par déplacement du réceptacle respectif ou du support relativement à l'élément de retrait (16).

2. Inhalateur selon la revendication 1, dans lequel le support est guidé entre les deux déflecteurs (8) dans chaque cas avec un rayon de courbure au moins sensiblement constant.

3. Inhalateur selon la revendication 1 ou 2, **caractérisé en ce que** le support s'étend exclusivement entre les deux déflecteurs (8), et/ou **en ce que** le support est guidé entre les deux déflecteurs (8), au moins sensiblement le long d'un arc extérieur et intérieur, notamment d'un arc de cercle.

4. Inhalateur selon l'une des revendications précédentes, **caractérisé en ce que** les déflecteurs (8) dévient le support sur au moins 160° chacun, particulièrement au moins sensiblement 180° ou plus chacun.

5. Inhalateur selon l'une des revendications précédentes, **caractérisé en ce que** les portions du support entre les deux déflecteurs (8) sont guidées à un espacement au moins sensiblement constant l'une de l'autre.

6. Inhalateur (1) selon l'une des revendications précédentes,
avec un dispositif d'acheminement (5) qui comprend une pluralité de roues (12) pour pas à pas faire progresser et/ou pour guider le support,
dans lequel les roues (12) ont le même diamètre, sont agencées sur un rayon commun, peuvent être entraînées par un moyen d'entraînement commun, notamment une roue solaire (14), et/ou ont le même sens de rotation.

7. Inhalateur selon la revendication 6, **caractérisé en ce que** le diamètre des roues (12) correspond sensiblement à l'espacement des portions, dans lequel les portions du support entre les deux déflecteurs (8) sont guidées à un espacement au moins sensiblement constant l'une de l'autre.

8. Inhalateur (1) selon l'une des revendications précédentes,
avec un dispositif d'acheminement (5) ayant un engrenage, notamment un engrenage planétaire (21), pour faire progresser le support, le dispositif d'acheminement (5) ayant embrayage à roue libre (22), de préférence dans lequel l'embrayage à roue libre (22) est agencé en une roue d'entraînement et/ou une roue d'engrenage, notamment en une roue solaire (14), et/ou de préférence dans lequel le dispositif d'acheminement (5) a un mécanisme anti-retour (28) qui forme un limiteur de course pour le mouvement arrière du support, particulièrement pour être capable de positionner le réceptacle respectif dans une position définie pour l'administration suivante.

9. Inhalateur selon l'une des revendications précédentes, **caractérisé en ce que** le dispositif d'acheminement (5) a en particulier uniquement deux ou quatre roues (12) qui agissent sur le support, et/ou **en ce que** les roues (12) agissant sur le support sont entraînées, et/ou **en ce que** le dispositif d'acheminement (5) forme l'unique entraînement pour déplacer le support.

10. Inhalateur selon l'une des revendications précédentes, **caractérisé en ce que** l'inhalateur (1) a un embout buccal (7) et un couvercle associé (15) qui est déplaçable ou pivotable pour ouvrir et/ou fermer l'embout buccal (7), dans lequel l'inhalateur (1) est construit de manière à ce que le dispositif d'acheminement (5) et/ou le dispositif de retrait (6) peuvent être actionnés ou entraînés pour ouvrir les réceptacles individuels et/ou retirer les doses de formulation (2) au moyen du mouvement d'ouverture et/ou de fermeture du couvercle (15).

11. Inhalateur selon l'une des revendications précédentes, **caractérisé en ce que** l'élément de guidage (13) est mobile à la manière d'un traineau et/ou à angles droits par rapport à la direction d'acheminement ou de progression du support.

12. Inhalateur selon l'une des revendications précédentes, **caractérisé en ce que** l'élément de retrait (16) est construit comme un élément de perforation, formé de manière fixe et/ou attaché sur un embout buccal (7) de l'inhalateur (1).

13. Inhalateur (1) selon l'une des revendications précédentes, dans lequel l'élément de retrait (16) est au moins sensiblement stationnaire, et dans lequel
le support est mobile à angles droits par rapport à la direction de progression en direction de l'élément de retrait (16) de manière à ouvrir le support ou un réceptacle du support ou à se raccorder à l'élément de retrait (16).

14. Inhalateur (1) selon l'une des revendications précédentes,
avec un boîtier sensiblement rond (19) qui est aplati ou indenté sur un côté, de préférence dans lequel le côté aplati ou indenté est formé par une portion (29) du boîtier (19) et par une portion (30) d'un couvercle (15) de l'inhalateur (1) qui est adjacente à ladite portion (29) lorsque l'inhalateur est fermé.

15. Inhalateur selon l'une des revendications précédentes, **caractérisé en ce que** l'inhalateur (1) est de construction portative.
